## Europäisches Patentamt

**European Patent Office**

**Office européen des brevets**

(11) Veröffentlichungsnummer: **0 427 861 A1**

(12) **EUROPÄISCHE PATENTANMELDUNG**
veröffentlicht nach Art. 158 Abs. 3
EPÜ

(21) Anmeldenummer: 90905347.2

(22) Anmeldetag: 18.12.89

(86) Internationale Anmeldenummer:
PCT/SU89/00321

(87) Internationale Veröffentlichungsnummer:
WO 90/14117 (29.11.90 90/27)

(51) Int. Cl.⁵: **A61M 5/32**

(30) Priorität: 16.05.89 SU 4684320

(43) Veröffentlichungstag der Anmeldung:
22.05.91 Patentblatt 91/21

(84) Benannte Vertragsstaaten:
BE CH DE FR GB IT LI SE

(71) Anmelder: POLTAVSKY MEDITSINSKY
STOMATOLOGICHESKY INSTITUT
ul. Shevchenko, 23,
Poltava 314000(SU)

(72) Erfinder: MAZURIK, Sergei Mikhailovich
ul. Lenina, 92-57
Poltava, 314022(SU)
Erfinder: TSYGANOVA, Nina Alexeevna
ul. Lenina, 92-57
Poltava, 314022(SU)

(74) Vertreter: Nix, Frank Arnold, Dr.
Kröckelbergstrasse 15
W-6200 Wiesbaden(DE)

(54) INJEKTIONSNADEL IN EINER INDIVIDUELLEN, STERILEN HÜLLE SOWIE SPRITZE MIT EINER SOLCHEN NADEL FÜR DEN EINMALIGEN GEBRAUCH.

(57) Die Injektionsnadel in einer individuellen sterilen Umhüllung ist als Stab (1) mit einem durchgehenden Axialkanal (2) ausgebildet, der in einer Kanüle (3) befestigt ist. Der Stab (1) ist versehen mit einer Schutzumhüllung (4), die in Gestalt einer Röhre ausgebildet und unmittelbar auf diesen aufgesetzt ist, und mit einer Schutzkappe (5), die auf den Stab (1) mit der Schutzumhüllung (4) aufgesetzt ist und an der Kanüle (3) befestigt ist.

Bei einer Einmalspritze mit einer Nadel mit Schutzumhüllung (4) und Schutzkappe (5) ist diese mit der Einmalspritze mittels einer unlösbaren Verbindung verbunden.

Die Erfindung kann Anwendung finden für Injektionen in beliebigen medizinischen Anstalten und im privaten Gebrauch.

FIG.3

FIG.1

EP 0 427 861 A1

# INJEKTIONSNADEL IN EINER INDIVIDUELLEN STERILEN UMHÜLLUNG UND EINMALSPRITZE MIT DIESER NADEL

Technisches Gebiet

Die Erfindung bezieht sich auf die Medizintechnik, und zwar auf Injektionsnadeln in einer individuellen sterilen Umhüllung, sowie auf Einmalspritzen für Injektionen unter Verwendung solcher Nadeln.

Früherer Stand der Technik

Weit bekannt ist eine Injektionsnadel in einer individuellen sterilen Umhüllung in Gestalt eines Stabes mit einem durchgehenden Axialkanal, welcher Stab an einem Ende zugespitzt ist und mit dem anderen Ende an einer Kanüle befestigt ist, die zur Befestigung am Zylinder der Spritze eingerichtet ist, und die mit einer abnehmbaren Schutzkappe versehen ist, deren Innendurchmesser wesentlich größer ist als der Durchmesser des Stabes (beispielsweise die Injektionsnadeln aus der Produktion der Firma Trumo Europe, Belgien).

Die Kanüle der Nadel wird am Zylinder der Spritze befestigt, wonach das Injektionsmittel aus einer Ampulle oder einer Flasche aufgezogen wird. Dabei geht die Sterilität der Nadel, mittels derer das Injektionsmittel aufgezogen wird, am Rand der Ampulle oder des Flaschenpfropfens verloren. Vor dem Einführen des Mittels wird die entsterilisierte Nadel durch eine sterile ersetzt und danach wird die Injektion durchgeführt. Die Notwendigkeit des Wechsels der Nadel bedingt die Verwendung von zwei Nadeln bei der Injektion, was ökonomisch ungünstig ist.

Die Notwendigkeit der Verwendung von zwei Nadeln führt auch dazu, daß selbst bei einer Zerstörung der Einmalspritze eine wiederholte Verwendung der Nadeln wahrscheinlichist. Dies kann durch Unachtsamkeit oder mangelnde Gewissenhaftigkeit des medizinischen Personals geschehen, aber auch bei der Durchführung der Injektionen durch von Rauschmitteln oder Alkohol berauschten Personen. Bei einer solchen Verwendung der Injektionsnadeln kommt es zu einer Ansteckung durch den AIDS-Virus, einer Infektions- oder anderer Krankheiten.

Offenbarung der Erfindung

Der Erfindung liegt die Aufgabe zugrunde, eine Injektionsnadel in einer individuellen sterilen Umhüllung mit einer solchen Ausbildung der Umhüllung zu schaffen, daß ohne Verlust der Sterilität eine Nadel zum Aufziehen des Mittels und zur Injektion verwendet werden kann, wodurch die Notwendigkeit eines Wechsels der Nadel bei der Injektion vermieden wird und eine solche Nadel bei Einmalspritzen unter Ausschluß der Möglichkeit einer wiederholten Verwendung angewandt werden kann.

Die gestellte Aufgabe wird dadurch gelöst, daß eine Injektionsnadel in einer individuellen sterilen Umhüllung, die in Gestalt eines Stabes mit einem durchgehenden Axialkanal ausgeführt ist, der an einem Ende zugespitzt ist und mit dem anderen Ende in einer Kanüle befestigt ist, die zur Befestigung am Zylinder der Spritze eingerichtet ist, und die mit einer abnehmbaren Schutzkappe versehen ist, deren Innendurchmesser wesentlich größer als der Durchmesser des Stabes ist, erfindungsgemäß eine Schutzumhüllung vorgesehen ist, die unmittelbar auf den Stab aufgesetzt ist und eine Röhre darstellt, deren Innendurchmesser im wesentlichen gleich dem Durchmesser des Stabes ist und deren Länge die Länge des Stabes übersteigt.

Bei einer Injektionsnadel, die zum Aufziehen des Mittels aus Ampullen bestimmt ist, ist als Schutzumhüllung zweckmäßigerweise eine Röhre aus elastischem Material verwendet.

Bei einer Injektionsnadel, die zum Aufziehen des Mittels aus einer Flasche bestimmt ist, wird als Schutzumhüllung wünschenswerterweise eine Röhre aus hartem Werkstoff verwendet, die am seitens des zugespitzten Nadelendes gelegenen Ende eine Zuspitzung aufweist.

Die gestellte Aufgabe wird auch dadurch gelöst, daß an einer Einmalspritze für Injektionen eine Nadel verwendet wird, wie sie vorstehend beschrieben ist und die an der Einmalspritze mittels einer unlösbaren Verbindung befestigt ist.

Die Injektionsnadel in einer individuellen sterilen Umhüllung gemäß der vorliegenden Erfindung erlaubt das Aufziehen des Mittels ohne Verlust der Sterilität, was seinerseits das Aufziehen des Mittels und dessen Einführung mittels der gleichen Nadel ohne einen Wechsel ermöglicht, was einerseits Nadeln einspart und andererseits eine Verbindung der erfindungsgemäßen Nadel mit einer Einmalspritze durch eine unlösbare Verbindung gestattet und damit die Möglichkeit einer wiederholten Verwendung ausschließt.

Kurzbeschreibung der Zeichnungen

Nachfolgend wird die Erfindung durch die Beschreibung von konkreten Ausführungsbeispielen an Hand der beigegebenen Zeichnungen erläutert. In diesen zeigt:

Fig. 1 eine Injektionsnadel in einer individuellen sterilen Umhüllung gemäß der Erfindung im Längsschnitt;

Fig. 2 eine Ausführungvariante der Schutzumhüllung aus einem harten Werkstoff in einer perspektivischen Ansicht;

Fig. 3 eine Injektionsnadel in einer individuellen sterilen Umhüllung gemäß der Erfindung, angebracht an der Spritze, in einer perspektivischen Ansicht;

Fig. 4 eine Spritze mit Nadel gemäß der Erfindung beim Aufziehen des Mittels aus einer Flasche in einer perspektivischen Ansicht.

Beste Ausführungsform der Erfindung

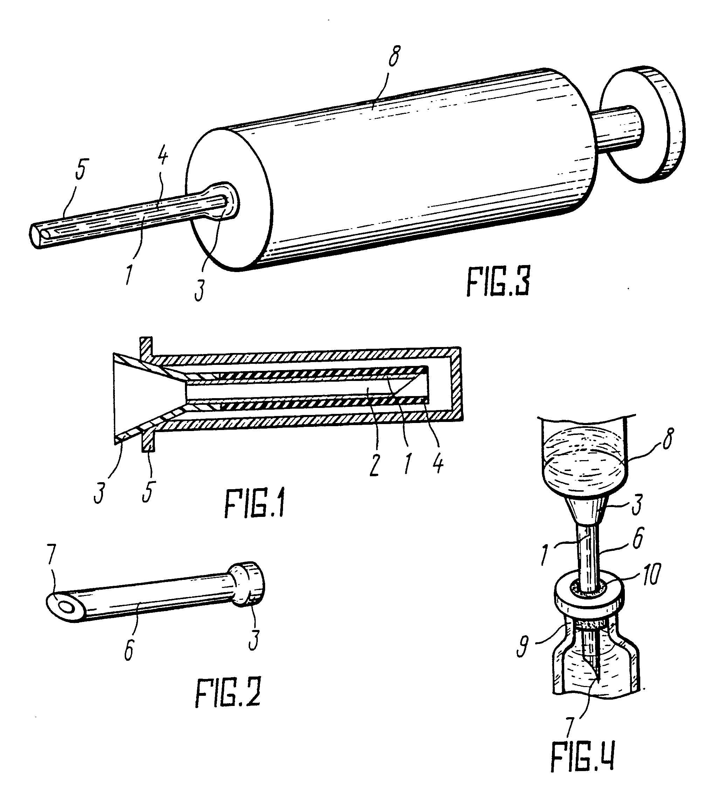

Die Injektionsnadel in einer individuellen sterilen Umhüllung ist ausgeführt in Gestalt eines Stabes 1 (Fig. 1) mit einem durchgehenden Axialkanal 2, der an einem Ende zugespitzt ist. Das andere Ende des Stabes 1 ist in einer Kanüle 3 befestigt, die zur Befestigung am Zylinder der Spritze (in Fig. 1 nicht gezeigt) eingerichtet ist.

Der Stab 1 ist mit einer Schutzumhüllung 4 versehen, die unmittelbar auf ihn aufgesetzt ist und eine Röhre darstellt, deren Innendurchmesser im wesentlichen gleich dem Durchmesser des Stabes 1 ist und deren Länge die Länge des Stabes 1 etwas überschreitet.

Außerdem ist die Nadel mit einer starren Schutzkappe 5 versehen, deren Durchmesser wesentlich größer als der Durchmesser des Stabes 1 ist.

Die Schutzkappe 5 hat eine innere Kegelfläche, die zur Zusammenwirkung mit der Kegeloberfläche der Kanüle 3 und zur Befestigung auf dieser bestimmt ist.

Bei einer Verwendung der erfindungsgemäßen Nadel zum Aufziehen des Mittels aus einer Ampulle ist die Schutzumhüllung 4 in Gestalt der Röhre aus einem elastischen Werkstoff, z.B. aus Gummi hergestellt.

Bei einer Verwendung der erfindungsgemäßen Nadel zum Aufziehen des Mittels aus einer Flasche, bei dem ein Durchstechen eines Gummipfropfens notwendig wird, ist die Schutzumhüllung 6 (Fig. 2) aus einem harten Werkstoff hergestellt, beispielsweise aus Polystyrol, und hat eine Zuspitzung 7 am Ende auf der Seite des zugespitzten Endes des Stabes.

Die Verbindung der Nadel mit der Spritze 8 (Fig. 3) kann sowohl trennbar als auch untrennbar (beispielsweise mit Klebstoff) ausgeführt sein. Bei einer Verwendung der erfindungsgemäßen Nadel an einer Einmalspritze wird sie mit dieser unlösbar verbunden.

Man gebraucht die erfindungsgemäße Injektionsnadel in einer individuellen sterilen Umhüllung wie folgt:

Vor dem Aufziehen des Injektionsmittels wird die Nadel mittels der Kanüle 3 (Fig. 3) an der Spritze 8 befestigt, wonach die Schutzkappe 5 abgenommen wird. Der Stab 1 in der Schutzumhüllung 4 wird in die geöffnete Ampulle eingeführt und danach zieht man das Mittel in die Spritze 8 auf.

Hierbei wird die Sterilität des Stabes 1 nicht beeinträchtigt, da beim Aufziehen des Mittels der Stab 1 sich in der sterilen Umhüllung 4 befindet und praktisch keinen Kontakt mit der Umgebung hat. Nach dem Aufziehen des Mittels wird die nach der Berührung mit der Ampulle nicht mehr sterile Schutzumhüllung 4 vom Stab 1 abgenommen, indem man sie an dem über den Stab 1 hinausragenden Ende ergreift. Die Injektion wird mit dem sterilen Stab 1 vorgenommen.

Beim Aufziehen des Mittels aus einer Flasche 9 (Fig. 4) wird eine Ausbildung der Injektionsnadel in einer individuellen sterilen Umhüllung verwendet, die eine Schutzumhüllung 6 aus hartem Werkstoff besitzt, welche eine Zuspitzung 7 am Ende seitens des zugespitzten Endes der Nadel aufweist. Die Steifigkeit des zugespitzten Endes 7 der Schutzumhüllung 6 ist derart, daß sie leicht durch einen Gummipfropfen 10 der Flasche 9 hindurchsticht.

Die weitere Reihenfolge der Manipulationen unterscheidet sich nicht von dem oben beschriebenen beim Aufziehen des Mittels aus einer Ampulle.

Bei einer Verwendung der erfindungsgemäßen Injektionsnadel, die unlösbar an einer Einmalspritze befestigt ist, ist die Möglichkeit ihrer wiederholten Verwendung ausgeschlossen, da sie gemeinsam mit der Einmalspritze entsorgt wird.

Die Schutzumhüllung 4 (6) gewährleistet die Sterilität der Nadel bei einer Weitergabe der Spritze mit aufgezogenem Mittel ohne Verwendung zusätzlicher Vorrichtungen (beispielsweise nierenförmiger Schalen).

Die breite Anwendung der Injektionsnadel in einer individuellen sterilen Umhüllung, die unlösbar mit einer Einmalspritze verbunden ist, kann praktisch Ansteckungen des Patienten bei Injektionen durch den AIDS-Virus, der infektiösen Hepatitis und anderer Krankheiten verhindern, wie sie bei parenteraler Applikation von Arzneimitteln übertragen werden.

Industrielle Anwendbarkeit

Die Injektionsnadel in einer individuellen sterilen Umhüllung kann zur Durchführung von subkutanen, intramuskulären und intravaskulären Injektionen in beliebigen medizinischen Anstalten sowie beim privaten Gebrauch Verwendung finden.

**Ansprüche**

1. Injektionsnadel in einer individuellen sterilen Umhüllung in Gestalt eines einen durchgehenden Axialkanal (2) aufweisenden Stabes (1), der an einem Ende zugespitzt ist, mit dem anderen Ende in einer Kanüle (3) befestigt ist und der mit einer abnehmbaren Schutzkappe (5) versehen ist, deren Innendurchmesser wesentlich größer als der Durchmesser des Stabes (1) ist,

   gekennzeichnet durch eine unmittelbar auf den Stab (1) aufgesetzte Schutzumhüllung (4) in Form einer Röhre, deren Innendurchmesser im wesentlichen gleich dem Durchmesser des Stabes (1) ist und deren Länge die Länge des Stabes (1) übersteigt.

2. Injektionsnadel nach Anspruch 1, dadurch gekennzeichnet, daß als Schutzumhüllung (4) eine Röhre aus elastischem Werkstoff verwendet ist.

3. Injektionsnadel nach Anspruch 1, dadurch gekennzeichnet, daß als Schutzumhüllung eine Röhre aus hartem Werkstoff verwendet ist, die am Ende auf der Seite des zugespitzten Endes der Nadel eine Zuspitzung (7) aufweist.

4. Einmalspritze für Injektionen, dadurch gekennzeichnet, daß an ihr die Nadel gemäß Anspruch 1 verwendet und mittels einer untrennbaren Verbindung befestigt ist.

**FIG.3**

**FIG.1**

**FIG.2**

**FIG.4**

# INTERNATIONAL SEARCH REPORT

International Application No PCT/SU 89/00321

## I. CLASSIFICATION OF SUBJECT MATTER (if several classification symbols apply, indicate all) 6

According to International Patent Classification (IPC) or to both National Classification and IPC

**IPC5:  A 61 M 5/32**

## II. FIELDS SEARCHED

### Minimum Documentation Searched 7

| Classification System | Classification Symbols |
|---|---|
| IPC4: | A 61 M 5/24, 5/32, 5/34 |

Documentation Searched other than Minimum Documentation
to the Extent that such Documents are Included in the Fields Searched 8

## III. DOCUMENTS CONSIDERED TO BE RELEVANT 9

| Category * | Citation of Document, 11 with indication, where appropriate, of the relevant passages 12 | Relevant to Claim No. 13 |
|---|---|---|
| A | FR, A1, 2366026, (OIWA SIGEO), 28 April 1978, the claims, figure 4 | 1,3 |
| A | EP, A1, 0201611, (INTERMEDI AT GMBH), 20 November 1986, the abstract | 1,3 |
| A | DE, C, 378629, (H. KOCH), 21 July 1923, the claims, the drawing | 1 |

* Special categories of cited documents: 10

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art.

"&" document member of the same patent family

## IV. CERTIFICATION

| Date of the Actual Completion of the International Search | Date of Mailing of this International Search Report |
|---|---|
| 24 May 1990 (24.05.90) | 6 July 1990 (06.07.90) |

| International Searching Authority | Signature of Authorized Officer |
|---|---|
| ISA/SU | |

Form PCT/ISA/210 (second sheet) (January 1985)